# EUROPEAN PATENT APPLICATION

(11) **EP 1 826 837 A1**
(43) Date of publication of application: **29.08.2007**
(21) Application number: 05805397.6
(22) Date of filing: 31.10.2005
(51) Int. Cl.: H01L 51/10, C07C 211/54, C09K 11/06

(54) **ORGANIC ELECTRONIC FUNCTIONAL MATERIAL AND USE THEREOF**

(30) Priority: 05.11.2004 JP 2004322782
(71) Applicant: Bando Chemical Industries, Ltd., Kobe-shi, Hyogo652-0883 (JP)
(72) Inventor: AKASHI, Nobutaka c/o Bando Chemical Industries, Lt, kobe-shi, Hyogo 652-0883 (JP); INAYAMA, Kaori c/o Bando Chemical Industries, Ltd., Hyogo 652-0883 (JP); INADA, Hiroshi c/o Bando Chemical Industries, Ltd., Hyogo 652-0883 (JP); SHIROTA, Yasuhiko, Toyonala-shi Osaka 561-0827 (JP)
(74) Representative: Cresswell, Thomas Anthony
(86) International application number: PCT/JP2005/020336
(87) International publication number: WO 2006/049271

(57) **Abstract**

The invention provides an organic electronic functional material which comprises a 1,3,5-tris(4-(N,N-diarylamino)phenyl)benzene represented by the general formula (I) 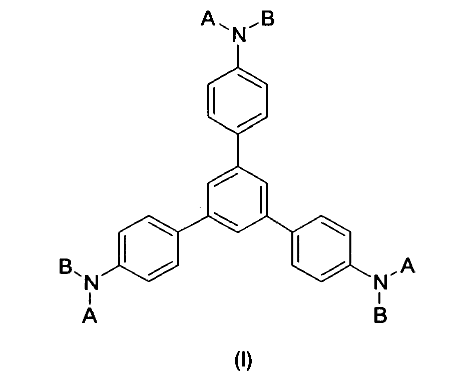 wherein A and B are each preferably a phenyl group having an alkyl group of 1-6 carbons or a cycloalkyl group of 5 or 6 carbons at the 4-position.

## Description

### Field of the Invention

This invention relates to an organic electronic functional material, and more particularly, to an organic electronic functional material which comprises a tris(4-(N,N-diarylamino)phenyl)benzene and is superior in repeated oxidation-reduction process and is hence suitable for use as, for example, a hole transporting agent in various electronic devices including an organic electroluminescence element.

### Background Art

In recent years, a variety of electronic devices such as organic semiconductors or light-emitting elements such as an electroluminescence elememt in which an organic compound which has photoelectric function as well as reversible oxidation-reduction characteristics and can form amorphous film by itself is used as an organic electronic material, for example, as a hole transporting agent, have attracted considerable attention, as described in JP-A-Nos. 6-1972 and 7-90256.

Such an amorphous film of organic substances are formed by preparing a coating composition comprised of a binder resin such as polycarbonate resin and the organic compound dissolved in a suitable organic solvent and then by coating and drying the composition, as described in JP-A-No. 11-174707. In the case where a nitrogen-containing polynuclear aromatic compound called a "star-burst" compound which is capable of forming amorphous film by itself, vacuum evaporation of the compound onto a substrate forms an organic amorphous film, as described in JP-A- No. 8-291115.

According to a method using a binder resin among the methods mentioned above, the organic compound is diluted with the binder resin in the resulting amorphous film and influenced by the binder resin so that the organic compound cannot exhibit sufficiently the functions that it originally has as an organic electronic functional material. In addition, if the organic compound forms an amorphous film that is stable at normal temperature with the aid of a binder resin, the organic compound has a low glass transition temperature so that the film is poor in heat resistance and has a problem in stability and life.

The nitrogen-containing polynuclear aromatic compounds called the "star-burst" molecules are divided into three groups based on their molecular structures: compounds having a triphenylamine structure (triphenylamines), compounds having a triaminobenzene structure (triaminobenzenes) and compounds having a triphenylbenzene structure (triphenylbenzenes).

Examples of the triphenylamines include, for example, 4,4',4"-tris- (N,N-diphenylamino) triphenylamine (TDATA) and 4,4',4"-tris(N-phenyl-N-m-tolylamino)triphenylamine (m-MTDATA), as described in JP-A-01-224353, and in addition, 4,4',4"-tris(N-(2-naphthyl)-N-phenylamino) triphenylamine (2-TNATA), as described in JP-A-08-291115.

These triphenylamines are reversible in oxidation-reduction process and can form amorphous film by a vacuum evaporation process, however, TDATA and m-MTDATA have a problem in heat resistance. On the other hand, TNATA has a glass transition temperature of about 110°C and is superior in heat resistance, but it is readily crystallized so that the amorphous film formed therewith is lacking in stability.

Examples of the triphenylbenzenes include, for example, 1,3,5-tris(4-(N,N-diphenylaminophenyl)benzene (TDAPB) and 1,3,5-tris(4-(N-tolyl-N-phenylaminophenyl)benzene (MTDAPB), as described in Bando Technical Report, Vol. 2, pp. 9-18, 1998 (Bando Chemical Industries, Ltd.). These triphenylbenzenes also can form amorphous film and have oxidation potentials in the range of 0.6-0.7V, but they are irreversible in oxidation-reduction process so that they are not suitable for practical use as an organic electronic functional material such as a hole transporting agent.

On the other hand, examples of the triaminobenzenes include 1,3,5-tris(N-methylphenyl-N-phenylamino)benzene (MTDAB). The triaminobenzenes also have oxidation potentials in the range of 0.6-0.7V, but they are irreversible in oxidation-reduction process so that they are also not suitable for practical use as an organic electronic functional material.

Further, 1,3,5-tris(N-(p-methylphenyl)-N-(-1-naphthyl))aminobenzene (p-MTPNAB) and 1,3,5- tris(N-(p-methylphenyl)-N-(4-biphenyl)amino)benzene (p-MTPBAB) have been proposed as such organic compounds that are reversible in oxidation-reduction process, have oxidation potentials in the range of 0.5-0.7V, are superior in heat-resistance, and can form amorphous film by a vacuum evaporation process, as described in JP-A-No.2004-155754.

The above-mentioned p-MTPNAB and p-MTPBAB are reversible in oxidation-reduction process and have high oxidation potentials as well as high glass transition temperature, i.e., 87°C and 98°C, respectively. However, when they are subjected to repeated oxidation-reduction process, peak currents of oxidation curves tend to fall, and accordingly, there is a fear that they have not enough stability and durability for use as organic electronic functional material.

The invention has been completed to solve the problems involved in the known organic electronic functional materials as mentioned above. Therefore, it is an object of the invention to provide an organic electronic functional material which has a photoelectric function, is reversible in oxidation-reduction process, can form amorphous film by itself, and has a high glass transition temperature, and shows only a slight change of peak current when being subjected to repeated oxidation-reduction process, and is hence superior in stability. Such an organic electronic functional material is can be suitably used as a hole transporting agent in various electronic devices inclusive of, for example, an organic electroluminescence element, and the like.

### Disclosure of the Invention

The invention provides an organic electronic functional material comprising a 1,3,5-tris(4-(N,N-diarylamino)phenyl)benzene represented by the general formula (I) 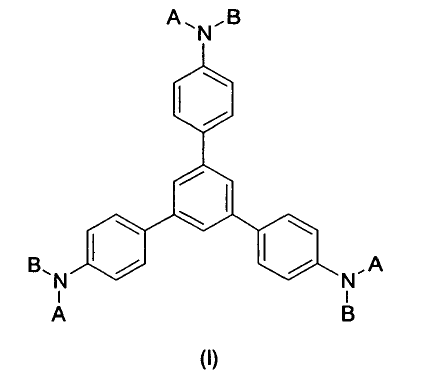 wherein A and B are each a group represented by the general formula (II) 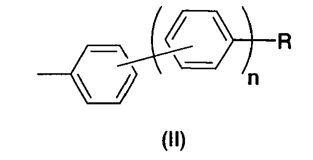 in which R represents an alkyl group of 1-6 carbons or a cycloalkyl group of 5 or 6 carbons; n is 0, 1, 2 or 3; and A and B may be the same or different from each other, and exhibiting a cyclic voltamogram in which a deviation of peak current of cyclic curves as measured 50 times at a sweep rate of 20 mV/s falls within ±10% of the average of peak current.

The invention further provides a hole transporting agent comprising the above-mentioned organic electronic functional material, and an organic electroluminescence element comprising a hole transporting layer comprising the hole transporting agent.

### Brief Description of the Drawings

Fig. 1 is a sectional view of an example of an organic electroluminescence element;
Fig. 2 is a differential scanning calorimetry (DSC) curve of 1,3,5-tris(4-(N,N-di-p-tolylamino)phenyl)benzene (p-DMTDAPB), one of the organic electronic functional materials of the invention;
Fig. 3 is a cyclic voltamogram of 1,3,5-tris(4-(N,N-dip-tolylamino)phenyl)benzene (p-DMTDAPB), one of the organic electronic functional materials of the invention;
Fig. 4 is a differential scanning calorimetry (DSC) curve of 1,3,5-tris(p-(N-phenyl-N-m-tolyl)aminophenyl)benzene (m-MTDAPB), one of the organic electronic functional materials of comparative examples;
Fig. 5 is a cyclic voltamogram of 1,3,5-tris(p-(N-phenyl-N-m-tolyl)aminophenyl)benzene (m-MTDAPB), one of the organic electronic functional materials of comparative examples;
FIG. 6 is a graph showing the time-luminance characteristic of an organic electroluminescence element having a hole transporting layer formed of a hole transporting agent, 1,3,5-tris(4-(N,N-di-p-tolylamino)phenyl)benzene (p-DMTDAPB), one of the organic electronic functional materials of the invention (Example 2) and the time-luminance characteristic of an organic electroluminescence element having a hole transporting layer formed of 1,3,5-tris(p-N-phenyl-N-m-tolyl)phenyl)benzene (m-MTDAPB), one of the hole transporting agents of comparative examples (Comparative Example 2);

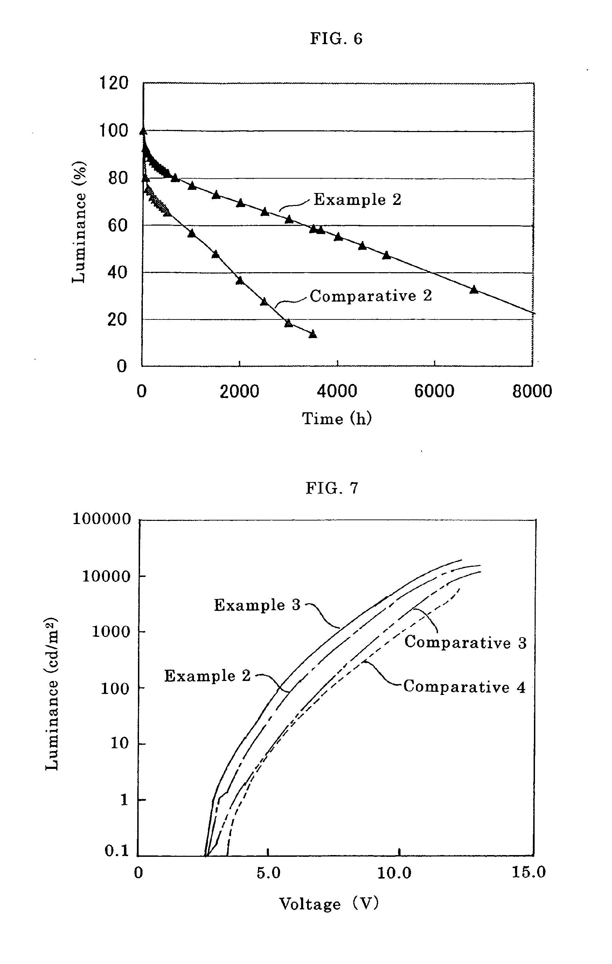

FIG. 7 is a graph showing the voltage-luminance characteristics of each of an organic electroluminescence element having a hole injecting layer formed of copper phthalocyanine and a hole transporting layer formed of an organic electronic functional material according to the invention (Example 2), an organic electroluminescence element having a hole injecting layer formed of 2-TNATA and a hole transporting layer formed of p-DMTDAPB, one of the organic electronic functional materials of the invention (Example 3), an organic electroluminescence element having a hole injecting layer formed of 2-TNATA and a hole transporting layer formed of 4,4'-bis[N-(1-naphthyl)-N-phenylamino]biphenyl (α-NPD) (Comparative Example 3), and an organic electroluminescence element having a hole injecting layer formed of copper phthalocyanine and a hole transporting layer formed of α-NPD) (Comparative Example 4).

### Best Mode of Carrying Out the Invention

The organic electronic functional material of the invention comprises a 1,3,5-tris(4-(N,N-diarylamino)phenyl)benzene represented by the general formula (I) 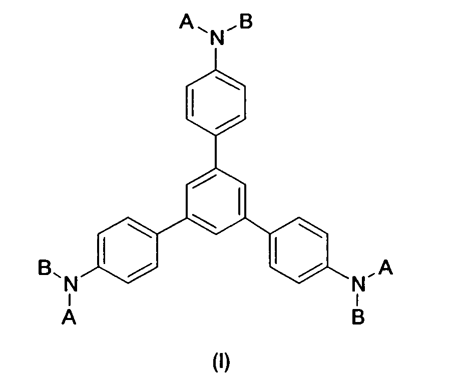 wherein A and B are each a group represented by the general formula (II) 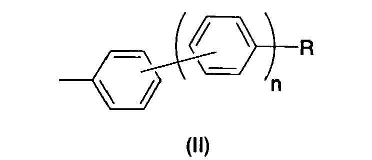 in which R represents an alkyl group of 1-6 carbons or a cycloalkyl group of 5 or 6 carbons; n is 0, 1, 2 or 3; and A and B may be the same or different from each other.

In the 1,3,5-tris(4-(N,N-diarylamino)phenyl)benzene represented by the general formula (I), the group A and B are each either a phenyl group having the alkyl or the cycloalkyl group, or a biphenylyl group having the alkyl or the cycloalkyl group at the terminal phenyl group, preferably a p-biphenylyl group, or a terphenyl group having the alkyl or the cycloalkyl group at the terminal phenyl group, preferably p- terphenylyl group, or a quaterphenyl group having the alkyl or the cycloalkyl group at the terminal phenyl group, preferably a p-quaterphenylyl group, and the group A and B may be the same or different from each other.

The 1,3,5-tris(4-(N,N-diarylamino)phenyl)benzene represented by the general formula (I) according to the invention is preferably such that the group A and B are each a phenyl group having the alkyl or the cycloalkyl group at the 4-position (or para-position) since such 1,3,5-tris (4-(N,N-diarylamino) phenyl) benzenes are excellent in the balance of reversibility of oxidation-reduction process, oxidation potential and heat resistance.

The alkyl group is methyl, propyl, butyl, pentyl or hexyl group, and may be linear or branched, and preferably methyl group, while the cycloalkyl group is cyclopentyl or cyclohexyl group.

Therefore, among the 1,3,5-tris(4-(N,N-diarylamino)phenyl)-benzenes, an organic electronic functional material comprising 1,3,5-tris(4-(N,N-di-p-tolylamino)phenyl)benzene represented by the formula (1) 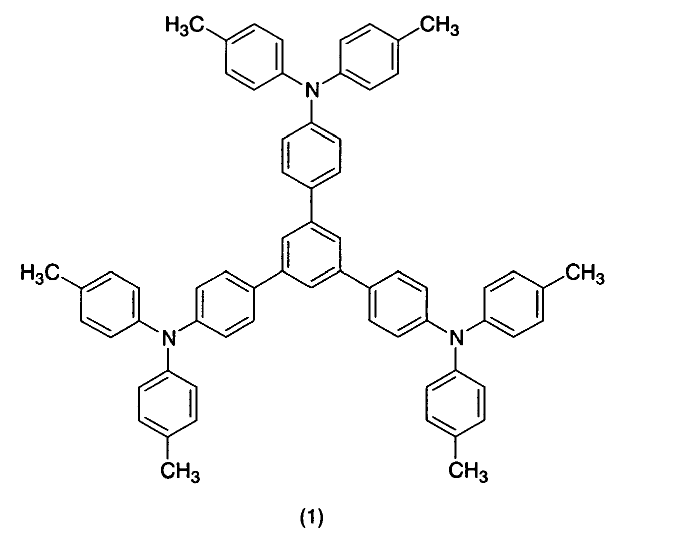 is superior in stability in repeated oxidation-reduction process and is suitable for use as a hole transporting agent in various electronic devices.

As shown in the scheme below, 1,3,5-tris(4-(N,N-di-p-tolylamino)phenyl)benzene can be obtained by, for example, reacting bis(4-tolyl)amine (2) with 1,3,5-tris(4-iodophenyl)benzene (3).

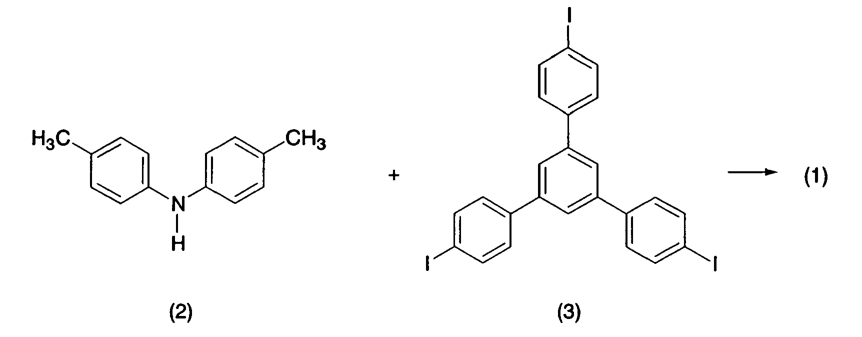

As described above, the organic electronic functional material of the invention comprises such a 1,3,5-tris(4-(N,N-diarylamino)-phenyl)benzene in which each of the chemically active sites at the terminal of the aryl group of the arylamino groups, preferably a carbon atom of para-position of phenyl group, is substituted or capped with a stable substituent such an alkyl or a cycloalkyl as mentioned hereinabove, so that the 1,3,5-tris(4-(N,N-diarylamino)phenyl)benzene, one of the "star-burst" molecules, is provided with improved reversibility in repeated oxidation-reduction process while retaining oxidation-reduction characteristics, high oxidation potential and high glass transition temperature. Accordingly, the organic electronic functional material of the invention shows only a slight change in the values of peak current in the repeated oxidation-reduction process, and can be used as a stable and durable organic electronic functional material in various electronic devices.

The organic electronic functional material comprising a 1,3,5-tris(4-(N,N-diarylamino)phenyl)benzene of the invention can form a stable amorphous film by itself at ordinary temperature or higher by a vacuum evaporation process, and moreover, it is excellent in reversibility in oxidation-reduction process, but also it has a high oxidation potential and a glass transition temperature. Therefore, the organic electronic functional material of the invention can be suitably used as a hole transporting agent in, for example, an organic electronic luminescence element.

The organic electroluminescence element is driven by a direct current at a low voltage with a high efficiency to emit light at a high luminance, as well as it can be made thin. Accordingly, in recent years, the investigation to put the organic electroluminescence element to practical use as display devices as well as backlights or illumination devices is pushed forward.

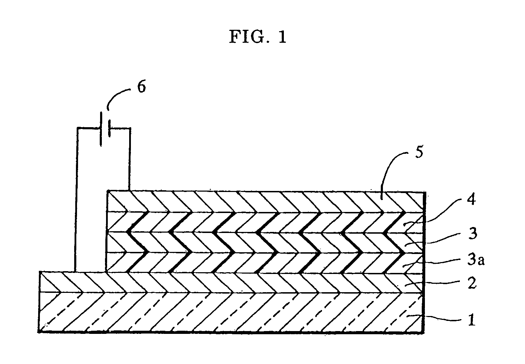

As an example is shown in Fig. 1, the electroluminescence element is comprised of a transparent substrate 1 made of glass, for example, having an anode 2 made of a transparent electrode such as an ITO membrane (indium oxide-tin oxide membrane) laminated thereon, and a hole injecting layer 3a, a hole transporting layer 3, an emitting layer 4 and a cathode 5 made of a metal or a compound thereof laminated on the anode in this order. The anode and the cathode are connected with an external power source 6. In some cases, a hole injecting layer 3a may be omitted, and an electron transporting layer may be laminated between the emitting layer and the cathode. An electroconductive polymer layer (a buffer layer) may be laminated between the anode and the hole transporting layer. Many other layer structures to form organic electroluminescence elements are already known.

The electroluminescence element of the invention is featured in that it has a hole transporting layer formed of a 1,3,5-tris(4-(N,N-diarylamino)phenyl)benzene represented by the general formula (I), or a hole transporting agent of the invention, but is not specifically limited in layer structures. The thickness of hole transporting layer (and a hole injecting layer) is usually in the range of 10 nm to 200 nm.

In such an organic electroluminescence element as mentioned above, the hole transporting layer adheres to the anode, and transports holes from the anode to the emitting layer while blocking electrons, whereas the electron transporting layer adheres to a cathode, and transports electrons from the cathode to the emitting layer. Thus, when an electron injected from the cathode and a hole injected from the anode recombine in the emitting layer, light is emitted and radiated outside through the transparent electrode (anode) and the transparent substrate.

In the organic electroluminescence element of the invention, the layers except the hole transporting layer mentioned above, that is, a transparent substrate, an anode, an emitting layer, an electron transporting layer and a cathode, may be made of any conventionally known materials. For example, an anode or transparent electrode may be made of indium oxide-tin oxide (ITO), and a cathode may be made of a metal such as aluminum, magnesium, indium or silver, or an alloy of these metals, such as Al-Mg alloy, Ag-Mg alloy, or a metal compound. A transparent substrate is usually made of glass.

The emitting layer is usually formed of tris(8-quinolinol) aluminum (Alq₃) and has a thickness in the range of 10 nm to 200 nm. The electron transporting layer has also a thickness in the range of 10 nm to 200 nm. When an electroconductive polymer layer is employed, it has also a thickness in the range of 10 nm to 200 nm.

When the organic electronic functional material of the invention is used as a hole transporting agent, a hole injecting layer that is formed of copper phthalocyanine (CuPC), a known hole injecting agent, may be placed between the anode and the hole transporting layer so that the energy gap between the anode and the hole transporting layer is made small and holes are readily transported from the anode to the hole transporting layer.

Further according to the invention, an organic electroluminescence element that is driven by a direct current at a lower voltage to emit light at a high luminance is obtained by using the organic electronic functional material of the invention as a hole transporting agent in conjunction with a tris(4-(N,N-diarylamino)-phenyl)amine represented by the general formula (III) 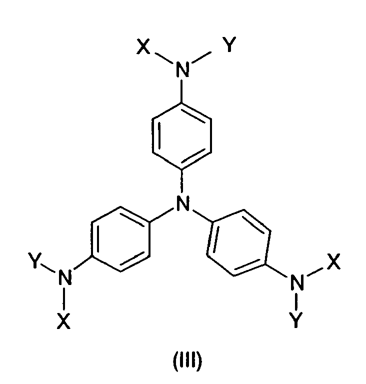 wherein X and Y are each an aryl group and may be the same or may be different from each other. That is, according to the invention, the voltage-luminance characteristics of an organic electroluminescence element is much more improved by laminating a hole injecting layer formed of the 1,3,5-tris (4-(N,N-diarylamino)phenyl)amine represented by the general formula (III) as a hole injecting agent and a hole transporting layer formed of the organic electronic functional material of the invention as a hole transporting agent.

If necessary, a uniform mixture of tris(4-(N,N-diarylamino)-phenyl)amine represented by the general formula (III) and the organic electronic functional material of the invention may be used as a hole transporting agent in manufacture of organic electroluminescence elements.

In the tris(4-(N,N-diarylamino)phenyl)amine represented by the general formula (III), X and Y are each an aryl group and may be the same or different from each other. Examples of such aryl groups include phenyl, o-, m- or p-tolyl, 1- or 2-naphthyl, 4-p-biphenylyl and 4-p-terphenylyl. Thus, Examples of the tris(4-(N,N-diarylamino)-phenyl)amine include, as mentioned hereinbefore, 4,4',4"-tris(N,N-diphenylamino)triphenylamine (TDATA), 4,4',4"-tris(N-phenyl-N-m-tolylamino)triphenylamine (m-MTDATA) and 4,4',4"-tris (N-(2-naphthyl)-N-phenylamino)triphenyl amine (2-TNATA), although the tris(4-(N,N-diarylamino)phenyl)amine usable are not limited to those exemplified.

### Industrial Applicability

The organic electronic functional material of the invention is comprised of 1,3,5-tris(4-(N,N-diarylamino)phenyl)benzene represented by the general formula (I), and has reversible oxidation-reduction characteristics as well as high oxidation potential and high glass transition temperature, and further it forms amorphous film stable at room temperature by itself by vacuum evaporation. In addition, the organic electronic functional material of the invention exhibits a deviation of peak current of cyclic curves as measured 50 times at a sweep rate of 20 mV/s falls within ±10%, and in a preferred embodiment, within ±5%, of the average of peak current in the measurement of voltamogram to illustrate the reversibility of oxidation-reduction process.

The organic electronic functional material of the invention is thus superior in reversibility in oxidation-reduction process so that it shows only slight change of peak current in repeated oxidation-reduction process and maintains the initial performance over a long period of time. Therefore, the organic electronic functional material of the invention is suitable for use as an organic electronic functional material such as a hole transporting agent in various electronic devices, for instance, in an organic electroluminescence element.

### Examples

The invention is described in more detail with reference to examples, however, the invention is not limited thereto.

### Example 1

### (Preparation of 1,3,5-tris(4-iodophenyl)benzene)

116 g of 1,3,5-triphenylbenzene, 19 mL of concentrated sulfuric acid as a catalyst, and 1520 mL of 80% acetic acid as a reaction solvent were placed in a 2 L capacity flask, and the mixture was heated to a temperature of 70°C with stirring. Then, 143 g of iodine and 69.3g of orthoperiodic acid were added 1/10 at a time over about 2 hours and a half into a flask, followed by reacting for 6 hours with stirring, to obtain a reaction product containing white precipitates.

Toluene was added to the reaction mixture to dissolve the precipitates therein, and the toluene layer was separated from the water layer. The toluene layer was washed with an aqueous solution of sodium hydrogencarbonate and then with an aqueous solution of sodium thiosulfate. The organic layer was then concentrated and subjected to silica gel chromatography and the reaction product was separated, which was recrystallized from ethanol/toluene/, thereby providing 34.6g of desired 1,3,5-tris(4-iodophenyl)benzene as white needle crystals. The yield was 13.3%.

### (Synthesis of 1,3,5-tris(4-(N,N-di-p-tolylamino)phenyl)benzene (p-DMTDAPB))

22.0 g of 1,3,5-tris(4-iodophenyl)benzene, 25.4 g of bis(4-tolyl) amine, 89.0 g of potassium carbonate, 11.4 g of copper powder, and 160 mL of mesitylene as a reaction solvent were placed in a 500 mL capacity glass flask and the reaction was carried out at a temperature of 165°C for 56 hours under a nitrogen atmosphere. After the reaction, the resultant reaction mixture was extracted with toluene and the toluene solution was subjected to silica gel chromatography to fractionate the reaction product. The reaction product was purified by recrystallization and then by sublimation to provide 4.3 g of the desired 1,3,5-tris(4-(N,N-di-p-tolylamino)phenyl)benzene. The yield was 15.1%.

| Elemental analysis (%): | | | |
|---|---|---|---|
| | C | H | N |
| Calculated: | 88.85 | 6.44 | 4.71 |
| Measured: | 88.65 | 6.54 | 4.81 |

| | | | |
|---|---|---|---|
| Molecular weight by mass analysis: 891 (M⁺) | | | |

### Differential scanning calorimetry (DSC)

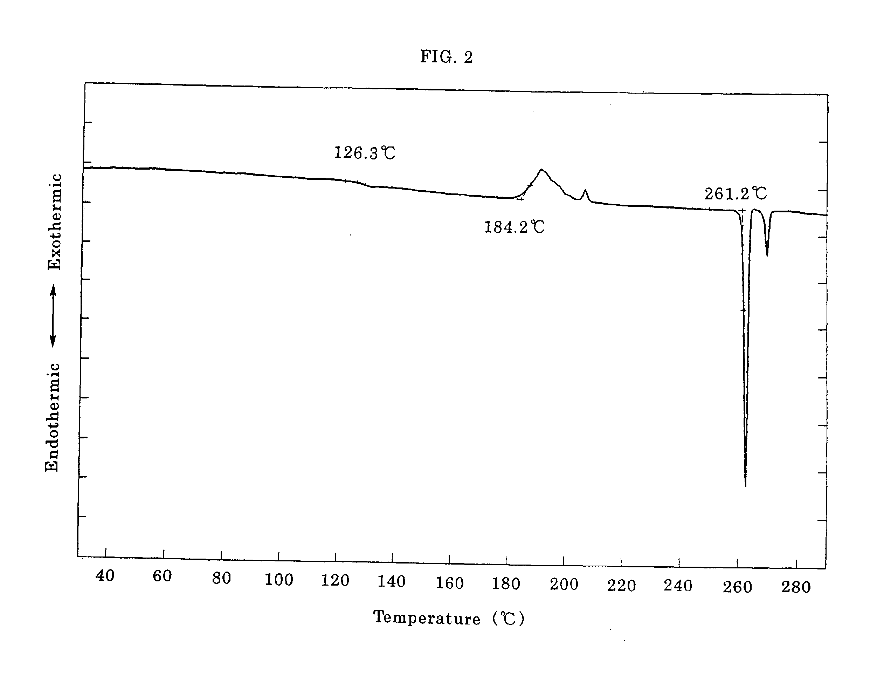

About 5 mg of p-DMTDABP was weighed as a sample, and it was melted in a differential scanning calorimetric device and then rapidly cooled in liquid nitrogen to form amorphous glass. Subsequently, the thermal characteristics of the sample were measured by heating at a rate of 5°C per minute by using an aluminum plate as a reference. As the DSC chart is shown in Fig. 2, the compound was found to have a glass transition temperature (Tg) of 126.3°C, a crystallization temperature (Tc) of 184.2°C, and a melting point of 261.2°C.

### Cyclic voltammetry (CV):

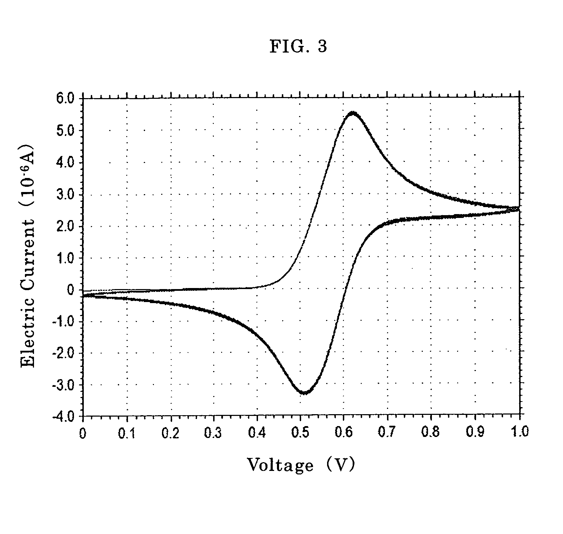

p-DMTDABP was dissolved in dichloromethane and the solution was arranged at a concentration of 10⁻³ M. The oxidation-reduction characteristics of the sample were measured using tetrabutylammonium perchlorate ((n-C₄H₉)₄NClO₄ (0.1M)) as a supporting electrolyte and Ag/Ag⁺ as a reference electrode at a scan speed of 20 mV/s. As the CV chart is shown in Fig. 3, the oxidation potential defined as an average of peak potential of oxidation curve and peak potential of reduction curve is 0.56 V (vs. Ag/Ag⁺), and the oxidation-reduction process was found to be reversible in 50 times measurement. In addition, the peak current of oxidation curve had an average of 5.488 x 10⁻⁶ A, a maximum of 5.563 x 10⁻⁶ A and a minimum of 5.413 x 10⁻⁶ A , and hence the deviation was only within ± 1.37 %. Thus, p-DMTDABP had stable oxidation-reduction characteristics and was almost free from deterioration in repeated oxidation-reduction process.

### Comparative Example 1

### (Synthesis of 1,3,5-tris(p-(N-phenyl-N-m-tolyl)aminophenyl)benzene (m-MTDAPB))

15.0 g of 1,3,5-tris(4-iodophenyl)benzene, 16.1 g of N-m-tolyl-N-phenylamine, 60.6 g of potassium carbonate, 7.8 g of copper powder, and 130 mL of mesitylene as a reaction solvent were placed in a 500 mL capacity glass flask and the reaction was carried out at a temperature of 165°C for 38 hours under a nitrogen atmosphere. After the reaction, the resultant reaction mixture was extracted with toluene and the toluene solution was subjected to silica gel chromatography to fractionate the reaction product. The reaction product was purified by recrystallization from toluene/ethanol and then by sublimation to provide 2.3 g of the desired 1,3,5-tris (p-N-phenyl-N-m-tolyl)aminophenylbenzene (m-MTDAPB). The yield was 10.5%.

| Elemental analysis (%): | | | |
|---|---|---|---|
| | C | H | N |
| Calculated: | 89.01 | 6.05 | 4.94 |
| Measured: | 89.31 | 5.98 | 4.71 |

| | | | |
|---|---|---|---|
| Molecular weight by mass analysis: 850 (M⁺) | | | |

### Differential scanning calorimetry (DSC):

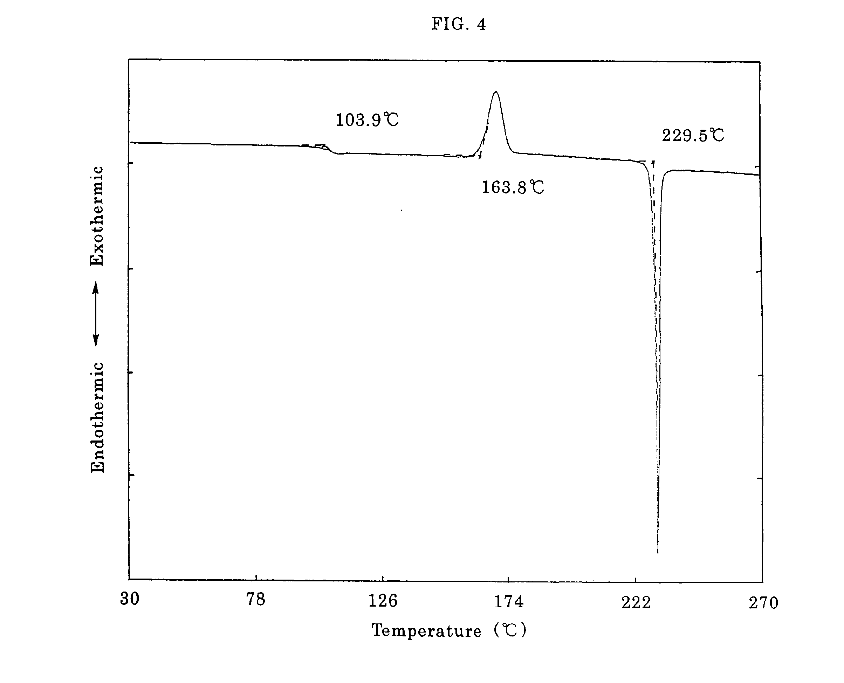

About 5 mg of m-MTDAPB was weighed as a sample, and it was melted in a differential scanning calorimetric device and then rapidly cooled in liquid nitrogen to form amorphous glass. Subsequently, the thermal characteristics of the sample were measured by heating at a rate of 5°C per minute by using an aluminum plate as a reference. As the DSC chart is shown in Fig. 4, the compound was found to have a glass transition temperature (Tg) of 103.9°C, a crystallization temperature (Tc) of 163.8°C, and a melting point of 229.5°C.

### Cyclic voltammetry (CV):

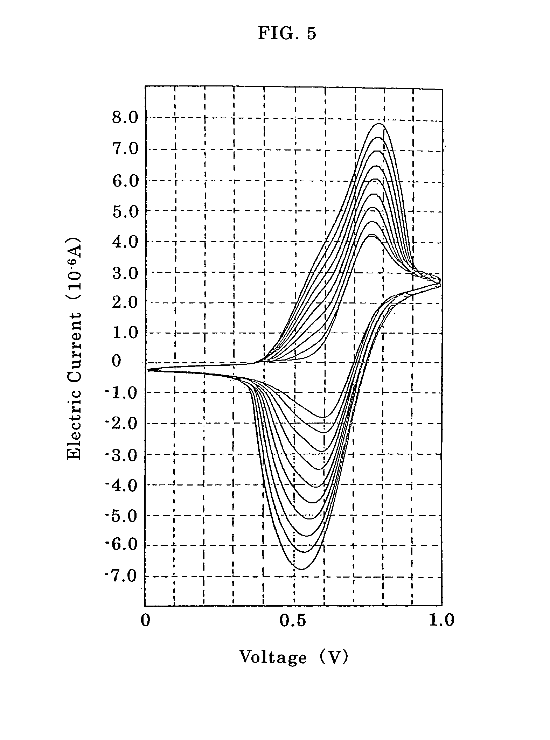

m-MTDAPB was dissolved in dichloromethane and the solution was arranged at a concentration of 10⁻³ M. The oxidation-reduction characteristics of the sample were measured using tetrabutylammonium perchlorate ((n·C₄H₉)₄NClO₄ (0.1M)) as a supporting electrolyte and Ag/Ag⁺ as a reference electrode at a scan speed of 100 mV/s. As the CV chart is shown in Fig. 5, the oxidation potential defined as an average of peak potential of oxidation curve and peak potential of reduction curve is 0.66 V (vs. Ag/Ag⁺). In repeated measurements, a new shoulder was observed and irreversibility was found in the oxidation-reduction process. The irreversibility of oxidation-reduction process is assumed to be derived from coupling reaction of radical cations. Moreover, it was found that the peak current of oxidation curve changed remarkably.

### Example 2

A sheet of plate glass having an ITO coating on one face (available from Sanyo Vacuum K.K.) was subjected to ultrasonic cleaning using acetone and then steam cleaning using methanol, followed by irradiation with ultraviolet rays by using a low-pressure mercury lamp for 10 minutes. Immediately after the irradiation, copper phthalocyanine (CuPC) was vacuum evaporated to form a hole injecting layer 20 nm thick and then p-DMTDAPB was vacuum evaporated to form a hole transporting layer 40 nm thick in this order on the ITO coating by using a vacuum evaporation apparatus. Subsequently, an emission layer 75 nm thick was formed of tris(8-quinolinol)aluminum (Alq₃) on the hole transporting layer, and then a lithium fluoride layer 0.5 nm thick and an aluminum layer 100 nm thick were layered in this order on the emission layer to form a cathode, thereby providing an organic electroluminescence element.

The change of luminance with time was examined by applying voltage across the electrodes of the organic electroluminescence element, letting the initial luminance (1000 cd/m²) be 100%. The results are shown in FIG. 6. The voltage-luminance characteristics of the organic electroluminescence element were also examined by applying voltage across the electrodes. The results are shown in FIG. 7.

### Comparative example 2

1,3,5-tris (p-N-phenyl-N-m-tolyl) aminophenylbenzene (m-MTDAPB) was used in place of p-DMTDAPB, and otherwise in the same manner as Example 2, an organic electroluminescence element was obtained. The change of luminance with time was examined by applying voltage across the electrodes of the organic electroluminescence element, letting the initial luminance (1000 cd/m²) be 100%.

As the results are shown in FIG. 6, the organic electroluminescence element having a hole transporting layer formed of p-DMTDAPB, a hole transporting agent of the invention, is superior in life to the organic electroluminescence element having a hole transporting layer formed of m-MTDAPB, a hole transporting agent of Comparative Example.

### Example 3

A sheet of plate glass having an ITO coating on one face (available from Sanyo Vacuum K.K.) was subjected to ultrasonic cleaning using acetone and then steam cleaning using methanol, followed by irradiation with ultraviolet rays by using a low-pressure mercury lamp for 10 minutes. Immediately after the irradiation, 4,4',4"-tris (N-(2-naphthyl)-N-phenylamino) triphenylamine (2-TNATA) was vacuum evaporated to form a hole injecting layer 50 nm thick and then p-DMTDAPB was vacuum evaporated to form a hole transporting layer 10 nm thick in this order on the ITO coating by using a vacuum evaporation apparatus. Subsequently, an emission layer 75 nm thick was formed of tris(8-quinolinol)aluminum (Alq₃) on the hole transporting layer, and then a lithium fluoride layer 0.5 nm thick and an aluminum layer 100 nm thick were layered in this order on the emission layer to form a cathode, thereby providing an organic electroluminescence element.

The voltage-luminance characteristics of the organic electroluminescence element were examined by applying voltage across the electrodes. The results are shown in FIG. 7.

### Comparative example 3

4,4'-bis[N-(1-naphthyl)-N-phenylamino] biphenyl (α-NPD) was used in place of p-DMTDAPB, and otherwise in the same manner as Example 3, an organic electroluminescence element was obtained. The voltage-luminance characteristics of the organic electroluminescence element were examined by applying voltage across the electrodes. The results are shown in FIG. 7.

### Comparative example 4

Copper phthalocyanine (CuPC) was used in place of 2-TNATA to form a hole injecting layer 20 nm thick and then α-NPD was used in place of p-DMTDAPB to form a hole transporting layer 40 nm thick on the hole injecting layer, and otherwise in the same manner as Example 3, an organic electroluminescence element was obtained. The voltage-luminance characteristics of the organic electroluminescence element was examined by applying voltage across the electrodes.

As the results are shown in FIG. 7, the organic electroluminescence elements each having the hole transporting layer formed of p-DMTDAPB, a hole transporting agent of the invention, and a hole injecting layer formed of the known hole injecting agent (Examples 2 and 3) had a higher luminance than the organic electroluminescence elements each having a hole transporting layer formed of the known hole transporting agent and a hole injecting layer formed of the known hole injecting agent (Comparative Examples 3 and 4) when the same voltage was applied.

## Claims

1. An organic electronic functional material comprising a 1,3,5-tris(4-(N,N-diarylamino)phenyl)benzene represented by the general formula (I) 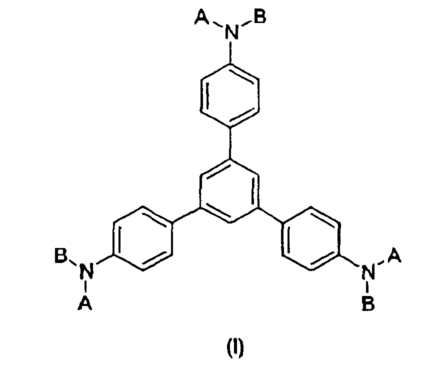 wherein A and B are each a group represented by the general formula (II) 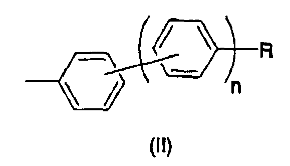 in which R represents an alkyl group of 1-6 carbons or a cycloalkyl group of 5 or 6 carbons; n is 0, 1, 2 or 3; and A and B may be the same or different from each other, and exhibiting a cyclic voltamogram in which a deviation of peak current of cyclic curves as measured 50 times at a sweep rate of 20 mV/s falls within ±10% of the average of peak current.

2. The organic electronic functional material as claimed in claim 1 which comprises a 1,3,5-tris(4-(N,N-diarylamino)phenyl)benzene wherein the groups A and B are each a phenyl group which has an alkyl group of 1-6 carbons or a cycloalkyl group of 5 or 6 carbons at the 4-position.

3. The organic electronic functional material as claimed in claim 1 which comprises 1,3,5-tris(4-(N,N-di-p-tolylamino)phenyl)-benzene.

4. A hole transporting agent comprising an organic electronic functional material as claimed in any one of claims 1 to 3.

5. An organic electroluminescence element which comprises a hole transporting layer comprising a hole transporting agent as claimed in claim 4

6. An organic electroluminescence element which comprises a hole transporting layer comprising a hole transporting agent as claimed in claim 4 and a hole injecting layer comprising a hole injecting agent comprising a tris(4-(N,N-diarylamino)phenyl)amine represented by the general formula (III) 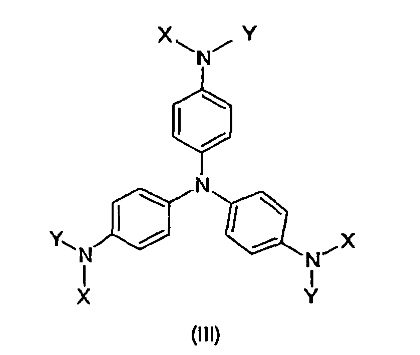 wherein X and Y are each an aryl group, and X and Y may be the same or different from each other.
